Europäisches Patentamt

European Patent Office

Office européen des brevets

(11) Veröffentlichungsnummer: **0 196 518**
**B1**

(12) **EUROPÄISCHE PATENTSCHRIFT**

(45) Veröffentlichungstag der Patentschrift: 27.12.90

(51) Int. Cl.⁵: **C 12 N 9/96,** G 01 N 33/535, C 12 Q 1/28, G 01 N 33/74, G 01 N 33/78, G 01 N 33/68, G 01 N 33/574

(21) Anmeldenummer: 86103434.6

(22) Anmeldetag: 14.03.86

(54) Stabilisierung der Aktivität von Peroxidase in Lösung.

(30) Priorität: 14.03.85 DE 3509238

(43) Veröffentlichungstag der Anmeldung:
08.10.86 Patentblatt 86/41

(45) Bekanntmachung des Hinweises auf die Patenterteilung:
27.12.90 Patentblatt 90/52

(84) Benannte Vertragsstaaten:
AT BE CH DE FR GB IT LI LU NL SE

(56) Entgegenhaltungen:
EP-A-0 029 926       EP-A-0 116 454
EP-A-0 054 358       US-A-4 252 896
EP-A-0 070 992       US-A-4 504 579
EP-A-0 103 784

CHEMICAL ABSTRACTS, Band 104, Nr. 17, 28. April 1986, Seite 538, Nr. 147015h, Columbus, Ohio, US

CHEMICAL ABSTRACTS, Band 95, Nr. 17, 26. Oktober 1981, Seite 258, Nr. 146156p, Columbus, Ohio, US

RESEARCH DISCLOSURE, Band 167, März 1978, Seiten 23-24, Nr. 16727, Havant, Hampshire, GB; "Reduction of gentisic acid interference in analytical elements"

(73) Patentinhaber: BOEHRINGER MANNHEIM GMBH
Patentabteilung, Abt. E Sandhofer Strasse 112-132 Postfach 31 01 20
D-6800 Mannheim 31 Waldhof (DE)

(72) Erfinder: Wehner, Rainer, Dr. rer. nat.
Buchendorfer Strasse 16
D-8035 Gauting (DE)
Erfinder: Mattersberger, Johann, Dr. phil.
Oefelestrasse 5
D-8000 München 90 (DE)
Erfinder: Klenner, Dagmar, Dr. rer. nat.
Höhenbergstrasse 1
D-8132 Tutzing (DE)
Erfinder: Deutsch, Gerlinde
Kapellenstrasse 12a
D-8139 Bernried (DE)

(74) Vertreter: Weickmann, Heinrich, Dipl.-Ing. et al
Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820
D-8000 München 86 (DE)

Courier Press, Leamington Spa, England.

**Beschreibung**

Peroxidase ist ein vielverwendetes Enzym, insbesondere im Rahmen enzymatischer Nachweisreaktionen. Auch im Rahmen der vielfältigen Ausgestaltungen von Enzymimmuntests (EIA) hat die Peroxidase als Markierungsenzym große Verbreitung gefunden. Peroxidasen verschiedener Herkunft, insbesondere die Meerrettich-Peroxidase, lassen sich mit einer Vielzahl von Methoden rasch und quantitativ nachweisen.

Hierauf beruht die sehr gute Eignung der Peroxidase als Nachweis- oder Markierungsenzym. Andererseits weist die Peroxidase (POD) jedoch den Nachteil auf, daß sie speziell in höherer Verdünnung und in Lösung eine nicht ganz befriedigende Aktivitätsstabilität aufweist. Die Lebensdauer insbesondere von Peroxidase-Konjugaten im Rahmen enzymatischer Testreagenzien oder anderer Enzympräparate ist daher beschränkt und um die angestrebte Lagerungsfähigkeit zu erzielen, ist eine Stabilisierung nötig.

Aus der DE—OS—31 00 076 ist es bereits bekannt, Peroxidase in einem Serumprotein enthaltenden Medium durch Zugabe von 8-Anilino-1-naphthalin-sulfonsäure (ANS) zu stabilisieren. Aus der EU—A—1 70 992 ist es bekannt, zur Stabilisierung der POD in einem Serum oder Serumprotein enthaltenden Medium 4-Aminoantipyrin zu verwenden. Aus der US—A—41 69 012 ist es schließlich bekannt, POD durch polyvalente Ionen aus den Gruppen 3 und 4 des periodischen Systems wie zum Beispiel Al, Zn, Mg, Fe und Cu zu stabilisieren. Diese bekannten Aktivitätsstabilisatoren vertragen sich mit anderen Zusätzen üblicher Reagenzkombinationen schlecht oder lassen hinsichtlich der Aktivitätsstabilisierung immer noch zu wünschen übrig. Außerdem beeinflussen sie teilweise die Immunreaktion negativ, wenn die POD als Konjugat mit einer immunologisch wirksamen Substanz vorliegt, was zu sehr flachen Eichkurven führt.

Der Erfindung liegt daher die Aufgabe zugrunde, die Aktivität von Peroxidase oder Peroxidase-Konjugaten in Lösung ohne die genannten Nachteile und ohne nachteilige Beeinflussung der eigentlichen Immunreaktion zu stabilisieren.

Gelöst wird diese Aufgabe erfindungsgemäß durch ein Verfahren zum Stabilisieren der Aktivität von Peroxidase-Konjugaten in Lösung durch Zusatz eines spezifischen Aktivitätsstabilisators, welches dadurch gekennzeichnet ist, daß man dem in fester oder gelöster Form vorliegenden Enzym-Konjugat mindestens 8 Stunden vor der Zugabe des Substrats als Aktivitätsstabilisator Phenol, das gegebenenfalls einen oder mehrere Substituenten trägt, die Niedrigalkylgruppen, Chlor- oder/und Bromatome sein können, in einer Menge von 0,0005 bis 2 Gew.-%, bezogen auf die Lösung, zusetzt.

Erfindungsgemäß wird die Enzymaktivität der frei oder gegebenenfalls auch in einem Konjugat kovalent gebunden vorliegenden Peroxidase gegen den besonders in verdünnten Lösungen sehr ausgeprägten inaktivierenden Einfluß von Temperatur, Fremdsubstanzen und dergleichen stabilisiert. Dies bedeutet, daß die in Lösung auftretende rasche Abnahme der Enzymaktivität beseitigt bzw. stark vermindert wird, ohne daß hierdurch die immunologische Wirksamkeit einer an die Peroxidase gebundenen, immunologisch wirksamen Substanz im Konjugat beeinträchtigt wird.

Als Aktivitätsstabilisator wird im Rahmen der Erfindung Phenol selbst oder ein Derivat desselben, welches mit einer oder mehreren Niedrigalkylgruppen, Chloratomen oder/und Bromatomen substituiert ist, verwendet. Bevorzugte Stabilisatoren sind neben Phenol selbst Bromphenol, Chlorphenol, Dichlorphenol, Dibromphenol und Kresol. Die Substituenten können hierbei in allen Positionen des aromatischen Rings stehen. Bevorzugt werden unter den substituierten Phenolen solche mit ein oder zwei Substituenten im Ring. Unter Niedrigalkylgruppen werden im Rahmen der Erfindung solche mit 1 bis 3 Kohlenstoffatomen verstanden.

Der erfindungsgemäße Aktivitätsstabilisator wird wie oben bereits erwähnt, in einer Menge zwischen 0,005 und 2 Gew.-%, bezogen auf das Volumen der Lösung, in der die POD enthalten ist, zugesetzt. Bei Überschreitung der Obergrenze von 2% bleibt zwar die Belastungsfähigkeit gegenüber Wärmeeinwirkung nach wie vor erhalten, die POD-Aktivität selbst wird jedoch wieder herabgesetzt. Unterhalb der unteren Grenze wird keine zufriedenstellende Stabilisierung mehr erzielt. Bevorzugt wird der Aktivitätsstabilisator gemäß der Erfindung in Mengen zwischen 0,01 und 0,5 Gew.-% zugesetzt.

Der Aktivitätsstabilisator kann dem in fester oder gelöster Form vorliegenden Enzym oder Enzymkonjugat zu einem beliebigen Zeitpunkt zugesetzt werden. Bevorzugt wird ein Zusatz zur Lösung, da hierdurch eine gleichmäßigere Verteilung des Stabilisators und damit eine bessere Wirkung auch bei Zusätzen an der unteren Grenze des wirksamen Bereiches erzielt wird. Insbesondere kann man das Phenol der Enzym- bzw. Konjugatlösung vor der Lyophilisierung oder nach dem Rekonstituieren des Lyophilisats mit einem wäßrigen Lösungsmittel zusetzen, wobei man im letzteren Fall natürlich auch das Lösungsmittel zuerst mit dem Aktivitätsstabilisator mischen und dann dem Lyophilisat zu dessen Auflösung zugeben kann.

Als immunologisch wirksame Substanz im Konjugat wird bevorzugt ein Antikörper oder ein Fragment davon, ein Antigen oder ein Hapten verwendet.

Als Antigene bzw. Haptane kommen beispielsweise in betracht Proteine, Drugs, Steroide und Hormone. Beispiele hierfür sind TBG, Cortisol, Vitamin $B_{12}$, Digoxin, Digoxigenin, Trijodthyronin und Thyroxin.

Als Antikörper kommen polyklonale und monoklonale Antikörper und deren Fragmente in betracht. Ebenfalls können chemisch abgewandelte Derivate derselben, beispielsweise mit Glutardialdehyd vernetzte Antikörper Komponenten des Konjugats darstellen. Beispiele hierfür sind Antikörper gegen TSH, hCG, AFP, LH, FSH, Prolactin, Ferritin, CEA und Insulin.

Als POD kommen alle Abarten dieses Enzyms in betracht. Bevorzugt wird aufgrund ihrer guten Zugänglichkeit die Meerrettich-POD.

Zweckmäßig wird bei erfindungsgemäßen Verfahren neben dem erwähnten Aktivitätsstabilisator noch ein Konservierungsmittel zugesetzt. Geeignete Konservierungsmittel sind zum Beispiel Merthiolat, Germall, Dowicil und Kathon CG.

Ein weiterer Gegenstand der Erfindung ist ein stabilisiertes substratfreies Enzympräparat mit einem Gehalt an Peroxidase-Konjugat, welches dadurch gekennzeichnet ist, daß es 0,005 bis 2 Gew.-%, bezogen auf seine gebrauchsfertige Lösung, Phenol, das gegebenenfalls einen oder mehrere Substituenten trägt, die Niedrigalkylgruppen, Chlor- oder/und Bromatome sein können, enthält.

Hinsichtlich der Komponenten des Konjugats, der Mengen an Aktivitätsstabilisator und eines etwaigen Gehaltes an zusätzlichem Konservierungsmittel gelten die obigen Ausführungen zum Verfahren für das stabilisierte Enzympräparat sinngemäß. Außerdem enthält das Enzympräparat zweckmäßig noch Puffersubstanzen, beispielsweise Phosphatpuffer, Citratpuffer, Boratpuffer und dergleichen oder/und Rinderserumalbumin oder/und ein System zum Nachweis der Peroxidaseaktivität.

Die Erfindung ermöglicht eine Stabilisierung der Enzymaktivität von POD oder POD-Konjugaten gegen die in Lösung normalerweise auftretende rasche Aktivitätsabnahme ohne nachteilige Beeinflussung der Immunreaktion bei den Konjugaten. Dabei gelingt erfindungsgemäß die Aktivitätsstabilisierung auch bei höheren Temperaturen über längere Zeit, so daß die Verwendbarkeit gebrauchsfertiger Lösungen von POD oder POD-Konjugaten wesentlich verbessert wird.

Die folgenden Beispiele erläutern dies näher.

## Beispiel 1

Stabilisierung von Antikörper-POD-Konjugat

| Lösung 1 | |
|---|---|
| Inkubationspuffer | |
| Phosphatpuffer | 15 mmol/l, pH 6,9 |
| Rinderserumalbumin (RSA) | 0,2 Gew.-% |
| Merthiolat | 0,01 Gew.-% |

| Lösung 2 | |
|---|---|
| Anti-TSH-POD-Konjugat | |
| gelöst in Lösung 1) | ca. 40 U/l |

| Lösung 3 | |
|---|---|
| Substrat/Puffer-Lösung | |
| Phosphat-Citrat-Puffer | 95 mmol/l, pH 4,4 |
| Natriumperborat | 3,1 mmol/l |
| 2,2'-Azino-di-[3-ethyl-benz- | |
| thiazolin-sulfonsäure (6)]- | |
| diammoniumsalz | 1,6 mmol/l |
| (ABTS®) | |

Die verwendeten Lösungen, beschichtete Röhrchen und Standard entstammen dem Enzymun-Test® TSH (Boehringer Mannheim GmbH, Best.-Nr. 73 60 83). Die Durchführung der Bestimmung erfolgt analog der Vorschrift des Herstellers.

In mit Anti-TSH-Antikörpern beschichteten Röhrchen werden 1 ml Lösung 1 und 0,2 ml TSH-Standard (ca. 50 μU/ml) gegeben und 60 Minuten bei 20 bis 25°C inkubiert. Nach Aussaugen und Spülen wird 1 ml Lösung 2 zugegeben. Die hierzu verwendete Lösung 2 wurde entweder frisch angesetzt (Vergleichsmessung) oder vor Zusatz 8, 18 bzw. 24 Stunden bei 30°C inkubiert, wobei gegebenenfalls 0,01 Gew.-% Phenol zugesetzt wurde.

Nach 60 Minuten Inkubation bei 20 bis 25°C werden die Röhrchen ausgesaugt und gespült. Anschließend wird 1 ml Lösung 3 zugegeben und wieder 60 Minuten bei 20 bis 25°C inkubiert. Danach wird gegen Lösung 3 als Leerwert eine photometrische Bestimmung bei λ=405 nm durchgeführt.

Die in der nachfolgenden Tabelle I aufgeführten Werte sind jeweils bezogen auf den relativen Wert 100%, der mit einem Test erhalten wurde, bei dem frisch angesetzte Lösung 2 (ohne Phenol) verwendet wurde.

Analoge Messungen wurden durchgeführt mit

| | |
|---|---|
| Enzymun-Test® Ferritin | Best.-Nr. 67 73 37 |
| Enzymun-Test® TBK | Best.-Nr. 24 94 16 |
| Enzymun-Test® Digoxin | Best.-Nr. 19 96 56 |
| Enzymun-Test® AFP | Best.-Nr. 71 14 11 |
| (Hersteller: Boehringer Mannheim GmbH) | |

3

# EP 0 196 518 B1

## TABELLE I

|  | 0 Std. |  | 8 Std. |  | 18 Std. |  | 24 Std. |  |
|---|---|---|---|---|---|---|---|---|
| Phenol 0,01 Gew.-% | + | − | + | − | + | − | + | − |
| Anti-TSH-POD | 100 | — | — | 90 | 21 | — | — | |
| Anti-Ferritin-POD | 100 | 97 | 62 | — | — | 90 | 40 | |
| T$_4$-POD (TBK-Test) | 100 | | | — | — | 70 | 4 | |
| Digoxin-POD | 100 | 99 | 77 | — | — | 93 | 48 | |
| Anti-AFP-POD | 100 | 96 | 89 | — | — | 86 | 70 | |

## Beispiel 2

Stabilisierung von freier Peroxidase mit Phenol/Phenolderivaten

Inkubationspuffer:
KH$_2$PO$_4$-Puffer    10 mmol/l, pH 7,5
NaCl    100 mmol/l
RSA    0,5 Gew.-%
Phenol bzw.
Phenolderivat    0,02 Gew.-%

POD wird im Inkubationspuffer 24 bzw. 48 Stunden bei 30°C belastet, anschließend die POD-Aktivität bestimmt und mit der Aktivität unbelasteter Lösungen verglichen. (Unbelastete Lösungen sind Lösungen, die 24 bzw. 48 Stunden bei 4°C gelagert werden).

Zur Bestimmung der POD-Aktivität wurden 0,1 ml Inkubationspuffer und 2,9 ml POD-Lösung bei 25°C gemischt, photometrisch bei λ=405 nm 5 Minuten lang die Extinktionsänderung verfolgt und E/min ermittelt. Die POD-Aktivität wird berechnet nach:

$$\text{POD-Aktivität} = \frac{100 \times 3}{0,1 \times 36,1 \times 1} \times \Delta E/\text{min} \ [\text{mU/ml}]$$

Als Ergebnis wurde Tabelle II und III erhalten.

## TABELLE II

| Stabilisator | POD-Aktivität (mU/ml) | |
|---|---|---|
|  | unbelastet | 48 Std./30°C |
| ohne Stabilisator | 117 | 43 |
| 0,02% Phenol | 130 | 130 |
| 0,02% 4-Bromphenol | 143 | 140 |
| 0,02% 4-Chlorphenol | 140 | 135 |
| 0,02% 2,3-Dichlorphenol | 130 | 120 |
| 0,02% 2,4-Dichlorphenol | 150 | 135 |
| 0,02% 2,5-Dichlorphenol | 130 | 125 |
| 0,02% 3,4-Dichlorphenol | 140 | 135 |
| 0,02% 3,5-Dichlorphenol | 140 | 125 |

# EP 0 196 518 B1

TABELLE III

| Stabilisator | POD-Aktivität (mU/ml) | |
|---|---|---|
| | unbelastet | 24 Std./30°C |
| ohne Stabilisator | 22,0 | 16,7 |
| 0,01% o-Kresol | 21,6 | 21,2 |
| 0,1% o-Kresol | 17,5 | 17,5 |
| 0,01% m-Kresol | 22,0 | 22,4 |
| 0,1% m-Kresol | 20,4 | 20,8 |
| 0,01% p-Kresol | 24,9 | 24,1 |
| 0,1% p-Kresol | 24,1 | 24,1 |

## Beispiel 3

Bei Versuchsdurchführung gemäß Beispiel 2 erhält man mit Phenolen verschiedener Konzentration Ergebnisse gemäß Tabelle IV.

TABELLE IV

| Stabilisator | POD-Aktivität (mU/ml) | |
|---|---|---|
| | unbelastet | 24 Std/30°C |
| 0,001% Phenol | 133 | 115 |
| 0,005% | 137 | 137 |
| 0,01% | 135 | 126 |
| 0,03% | 127 | 130 |
| 0,05% | 133 | 137 |
| 0,1% | 133 | 132 |
| 0,3% | 124 | 123 |
| 0,5% | 120 | 117 |
| 1,0% | 120 | 97 |
| 2,0% | 61 | 60 |
| 0,001% 4-Bromophenol | 140 | 138 |
| 0,005% " | 133 | 140 |
| 0,01% " | 139 | 140 |
| 0,03% " | 137 | 135 |
| 0,05% " | 145 | 143 |
| 0,1% " | 158 | 160 |
| 0,3% " | 185 | 177 |
| 0,5% " | 172 | 138 |
| 0,001% 2,4-Dichlorphenol | 130 | 129 |
| 0,005% " | 140 | 146 |
| 0,01% " | 137 | 137 |
| 0,03% " | 130 | 133 |
| 0,05% " | 143 | 145 |

0,5%: Pufferlösung mit 4-Bromphenol gesättigt.
0,05%: Pufferlösung mit 2,4-Dichlorphenol gesättigt.

## Patentansprüche

1. Verfahren zum Stabilisieren der Aktivität von Peroxidasekonjugaten in Lösung durch Zusatz eines spezifischen Aktivitätsstabilisators, dadurch gekennzeichnet, daß man einer Lösung des Peroxidasekonjugats, die kein Peroxidasesubstrat enthält, als Aktivitätsstabilisator Phenol, das gegebenenfalls einen oder mehrere Substituenten trägt, die Niedrigalkylgruppen, Chlor- oder/und Bromatome sein können, in einer Menge von 0,0005 bis 2 Gew.-%, bezogen auf die Lösung zusetzt und vor Zugabe des Substrats den Aktivitätsstabilisator wieder abtrennt.

2. Verfahren nach Anspruch 1, dadurch gekennzeichnet, daß man 0,01 bis 0,5 Gew.-% des Phenols zusetzt.

3. Verfahren nach Anspruch 1 oder 2, dadurch gekennzeichnet, daß man das Phenol der zur Lyophilisierung bestimmten Konjugatlösung zusetzt.

4. Verfahren nach Anspruch 1 oder 2, dadurch gekennzeichnet, daß man das Phenol dem mit wäßrigem Lösungsmittel rekonstituierten Lyophilisat zusetzt.

5. Verfahren nach einem der vorhergehenden Ansprüche, dadurch gekennzeichnet, daß die Peroxidase als Konjugat mit einer immunologisch wirksamen Substanz verwendet wird.

6. Verfahren nach Anspruch 5, dadurch gekennzeichnet, daß die immunologisch wirksame Substanz ein Antikörper oder ein Fragment davon, ein Antigen oder ein Hapten ist.

7. Verfahren nach Anspruch 6, dadurch gekennzeichnet, daß die immunologisch wirksame Substanz Digoxin, Digoxigenin, Trijodthyronin oder Thyroxin oder ein Antikörper gegen TSH, Ferritin oder AFP ist.

8. Verfahren nach einem der vorhergehenden Ansprüche, dadurch gekennzeichnet, daß man zusätzlich ein Konservierungsmittel zugibt.

9. Stabilisiertes substratfreies Enzympräparat mit einem Gehalt an Peroxidase-Konjugat, dadurch gekennzeichnet, daß es 0,005 bis 2 Gew.-%, bezogen auf seine gebrauchsfertige Lösung, Phenol, das gegebenenfalls einen oder mehrere Substituenten trägt, die Niedrigalkylgruppen, Chlor- oder/und Bromatome sein können, enthält.

**Revendications**

1. Procédé pour la stabilisation de l'activité des conjugués de peroxydase en solution par addition d'un stabilisant d'activité spécifique, caractérisé en ce qu'on ajoute au conjugué d'enzyme se présentant sous forme solide ou dissoute, au moins 8 heures avant l'addition du substrat comme stabilisant du phénol qui porte éventuellement un ou plusieurs substituants qui peuvent être des groupes alcoyle inférieur, des atomes de chlore ou/et de brome, en une quantité de 0,0005 à 2% en poids, par rapport à la solution.

2. Procédé selon la revendication 1, caractérisé en ce qu'on ajoute 0,01 à 0,5% en poids du phénol.

3. Procédé selon la revendication 1 ou 2, caractérisé en ce qu'on ajoute le phénol à la solution de conjugué destinée à la lyophilisation.

4. Procédé selon la revendication 1 ou 2, caractérisé en ce qu'on ajoute le phénol au lyophilisat reconstitué avec du solvant aqueux.

5. Procédé selon l'une des revendications précédentes, caractérisé en ce que la peroxydase est utilisée sous forme de conjugé avec une substance à activité immunologique.

6. Procédé selon la revendication 5, caractérisé en ce que la substance à activité immunologique est un anticorps ou un fragment de celui-ci, un antigène ou un haptène.

7. Procédé selon la revendication 6, caractérisé en ce que la substance à activité immunologique est la digoxine, la digoxigénine, la triiodothyronine ou la thyroxine ou un anticorps contre la TSH, la ferritine ou l'AFP.

8. Procédé selon l'une des revendications précédentes, caractérisé en ce qu'on ajoute en outre un conservateur.

9. Préparation d'enzyme sans substrat, stabilisée, présentant une teneur en conjugué de peroxydase, caractérisée en ce qu'elle contient 0,005 à 2% en poids, par rapport à sa solution prête à l'emploi, de phénol qui porte éventuellement un ou plusieurs substituants qui peuvent être des groupes alcoyle inférieur, des atomes de chlore ou/et des atomes de brome.

**Claims**

1. Process for the stabilising of the activity of peroxidase conjugate in solution by addition of a specific activity stabiliser, characterised in that to the enzyme conjugate present in solid or dissolved form, one adds, as activity stabiliser, at least 8 hours before the addition of the substrate, phenol which optionally carries one or more substituents which can be lower alkyl groups, chlorine and/or bromine atoms, in an amount of 0.0005 to 2 wt.%, referred to the solution.

2. Process according to claim 1, characterised in that one adds 0.01 to 0.5 wt.% of phenol.

3. Process according to claim 1 or 2, characterised in that one adds the phenol to the conjugate solution intended for the lyophilisation.

4. Process according to claim 1 or 2, characterised in that one adds the phenol to the lyophilisate reconstituted with aqueous solvent.

5. Process according to one of the preceding claims, characterised in that the peroxidase is used as conjugate with an immunologically-active substance.

6. Process according to claim 5, characterised in that the immunologically-active substance is an antibody or a fragment thereof, an antigen or a hapten.

7. Process according to claim 6, characterised in that the immunologically-active substance is digoxin, digoxigenin, triiodothyronine or thyroxin or an antibody against TSH, ferritin or AFP.

8. Process according to one of the preceding claims, characterised in that one additionally adds a preserving agent.

9. Stabilised, substrate-free enzyme preparation with a content of peroxidase conjugate, characterised in that it contains 0.005 to 2 wt.%, referred to its solution ready for use, of phenol which possibly carries one or more substituents which can be lower alkyl groups, chlorine and/or bromine atoms.